# EUROPEAN PATENT APPLICATION

(11) **EP 4 691 492 A2**
(43) Date of publication of application: **11.02.2026**
(21) Application number: 25208265.6
(22) Date of filing: 17.12.2019
(51) Int. Cl.: A61K 47/38

(54) **LIQUID COMPOSITIONS**

(30) Priority: 08.01.2019 SE 1950012
(62) Divisional of application: 19908975.6
(71) Applicant: Kenvue Brands LLC, Summit, NJ 07901 (US)
(72) Inventor: MCNALLY, Gerard P., Derwyn, 19312 (US); DAVE, Vipul, Fort Washington, 19034 (US); PANDEY, Anurag, Fort Washington, 19034 (US)
(74) Representative: Carpmaels & Ransford LLP

(57) **Abstract**

The present invention relates to a pharmaceutical liquid composition, wherein said liquid composition comprises at least one cooling agent comprising of a phenyl ring containing a polar side group at least one viscosity enhancing agent and at least one surfactant. The invention also relates to a method to produce such a pharmaceutical composition as well as a method of alleviating a symptom selected from the group consisting of cough, nasal congestion and sore throat in a subject comprising administering the composition according to any of preceding claims.

## Description

### FIELD OF THE INVENTION

The present invention relates to a pharmaceutical liquid composition, wherein said liquid composition comprises at least one cooling agent comprising a phenyl ring containing a polar side group, at least one viscosity enhancing agent and at least one surfactant. The invention also relates to a method of alleviating a symptom selected from the group consisting of cough, nasal congestion and sore throat in a subject comprising administering the composition.

### BACKGROUND OF THE INVENTION

A sore throat is characterized by a pain or irritation of the throat or pharynx, usually caused by acute pharyngitis. A sore throat is most often caused by a viral infection. A sore throat can also be caused by a streptococcal infection, tumors, gastroesophageal reflux disease, mononucleosis, and allergies.

A sore throat can develop for many reasons including a viral or bacterial infection, or a common or seasonal allergy. Often associated with an infection, common or seasonal allergy includes some degree of nasal or sinus congestion. This congestion is typically referred to as post-nasal drip, in which mucous originating on the surface of the nasal mucosa or the sinus mucosa drains onto the upper esophagus. The accumulation of nasal mucosa in the upper esophagus also stimulates the swallowing reflex often associated with a sore throat. The swallowing reflex transports the acidic mucous into relatively constant contact with the region of the throat. The acidic nature of the mucous from the sinus mucosa or nasal mucosa erodes the epithelial tissue of the throat thereby exposing the underlying tissue to the acidic mucous. The nerve endings in the underlying tissue in contact with the acidic mucosa cause what one identifies as the discomfort or pain associated with a sore throat. The more inflamed the nasal mucosa or the sinus mucosa, the greater the production of the acidic mucous, the greater the erosion and the greater the severity of the pain and discomfort associated with the sore throat.

The pain of sore throat can be treated with various dosage forms or remedies. Common dosage forms include throat sprays, lozenges, and orally administered tablets or liquids, all of which may contain active ingredients. Sprays and lozenges typically contain topical analgesics or menthol to cool the pain of a sore throat. Orally administered tablets or liquids typically contain systemically acting active ingredients for pain, cough and/or cold; including, e.g., acetaminophen, NSAIDs, decongestants, and/or cough suppressants. In some cases, these products contain sensates for cooling which also help in alleviating pain or providing the perception of alleviating pain.

There remains a need for liquid compositions and methods that are safe and effective to treat, soothe or reduce the severity of a sore throat. Such a composition should give a cooling effect, work quickly and provide superior sore throat relief for an extended period of time.

### SUMMARY OF THE INVENTION

The present invention is directed to pharmaceutical liquid compositions that can be used to alleviate sore throat. The compositions, which provide a thin layer of coating on oral surfaces, enhance the sensory experience, i.e., increase cooling effect as compared to known compositions. In particular, the composition provides longer lasting targeted cooling sensation in the throat region.

In a first aspect the invention relates to a pharmaceutical liquid composition, wherein said liquid composition comprises at least one cooling agent comprising of a phenyl ring containing a polar side group, at least one viscosity enhancing agent and at least one surfactant. The unique composition with the specific kind of cooling agent, viscosity agent and surfactant give rise to an improved mouth feel of the compositions, compared to when cooling agents having other structures in combination with the viscosity enhancing agent and surfactant are used. A cooling sensation is felt in the back of the throat as well as the sensation lasted longer compared to the other compositions.

Additional it was found that, the targeting effect and longer lasting sensation was not present in low viscosity liquids even when surfactant was included in the formulation. In presence of just the viscosity modifiers and no surfactant, the same flavor did provide targeted cooling, but the extent and duration was less which showed that the mixture of the viscosity agent, surfactant and the specific group of cooling agents gave rise to the effect of an improved mouth feel and a prolonged sensation.

The invention also relates to a method to produce such a pharmaceutical composition as well as a method of alleviating a symptom selected from the group consisting of cough, nasal congestion and sore throat in a subject comprising administering the pharmaceutical liquid composition.

The invention also relates to a method to produce such a pharmaceutical liquid composition as well as a method of alleviating a symptom selected from the group consisting of cough, nasal congestion and sore throat in a subject comprising administering the composition as disclosed herein.

### BRIEF DESCRIPTION OF THE DRAWINGS

Fig. 1 shows the Zeta potential of mucin particle in presence of CMC and Poloxamer-188.
Fig. 2 shows the steady state shear sweep curves for Poloxamer-188, CMC, Mucin and their combinations at 25 °C.
Fig. 3 shows the steady state shear sweep curves for 2 % Poloxamer-188, 1% CMC, 2% Mucin and their combinations at 25 °C.
Fig. 4 shows the steady state shear sweep curves for 2 % Poloxamer-188, 1% CMC, 2% Mucin and their combinations at 37 °C.
Fig. 5 shows the steady state shear sweep curves for 2 % Poloxamer-188, 1% HPMC, 2% Mucin and their combinations at 37 °C.
Fig. 6 shows 1H NMR of Poloxamer-188.
Fig. 7 shows 1H NMR of Cooler #2 (Monomethyl gluterate).
Fig. 8 shows 1H NMR of Cooler #2 (Monomethyl gluterate) and Poloxamer-188 in denaturated DMSO.
Fig. 9 shows conductivity measurement for Poloxamer Water system.
Fig. 10 shows conductivity measurement for Water-propylene glycol-Cooler #2 with 0,4 % Poloxamer-188.

### DETAILED DESCRIPTION OF INVENTION

### Definitions

In the context of the present application and invention the following definitions apply:
The term "active ingredient" is intended to encompass any ingredient that imparts a therapeutic effect. For example, the active ingredient can be both pharmaceutical and herbs.

The term "viscosity enhancing agent" or "thickening agent" is intended to mean any agent that can increase the viscosity of a liquid without substantially changing other properties.

The term "Rheology" means the study of the flow of matter, primarily in a liquid state, but also as "soft solids" or solids under conditions in which they respond with plastic flow rather than deforming elastically in response to an applied force. Rheology generally accounts for the behavior of non-Newtonian fluids, by characterizing the minimum number of functions that are needed to relate stresses with rate of change of strain or strain rates.

The term "Shear rheology" means the characterization of flow or deformation of a liquid originating from a simple shear stress field.

The term "Shear sweep" herein means the determination of the viscosity of a liquid at varied shear rates.

The term "Therapeutic effect" means any effect or action of an active ingredient intended to diagnose, treat, cure, mitigate, or prevent disease, or affect the structure or any function of the body.

The term "%w/w" is intended to mean the percentage of an ingredient(s)/the total percentage by weight of the composition (100 %).

### THE PHARMACEUTICAL LIQUID COMPOSITION

The invention relates to a pharmaceutical liquid composition, wherein said liquid composition comprises at least one cooling agent comprising of a phenyl ring containing a polar side group, at least one viscosity enhancing agent and at least one surfactant. By such as combination a synergetic effect in relation to the viscosity is achieved and the pharmaceutical liquid composition remains in the throat for a prolonged time and give a prolonged effect compared to other similar pharmaceutical liquid composition. In addition, by use of the specific group of cooling agents an increased sensation is achieved compared to with other cooling agents. The pharmaceutical liquid composition is improved in aiding a person suffering from cough, nasal congestion and sore throat by alleviating the symptoms. A prolonged cooling effect also aid in the sensation of treating sore throat when combined with therapeutic active ingredients.

The cooling agents may be selected from the group consisting of menthyl glutarate, menthyl lactate, N-ethyl 2-isopropyl-5-methylcyclohexanecarboxamide, N-[(ethoxycarbonyl)methyl)-p-menthane-3-carboxamide and (1R,2S,5R)-N-(4-methoxyphenyl)-5-methyl-2-(1-methylethyl)cyclohexanecarboxamide, wherein all of the cooling agents comprises a phenyl ring and a polar side group, preferably menthyl glutarate. In certain embodiments a combination of more than one cooler comprising a phenyl ring with a polar side group may be used. In one embodiment, a combination of a cooler comprising a phenyl ring with a polar side group and a cooler without a phenyl ring with a polar side group may be used. In another embodiment, the cooler comprising a phenyl ring with a polar side group is a cooler with a cyclohexyl core substituted at ring positions 1,3 and 4 and an ester or an amide group at one of the three positions.

The viscosity enhancing agent/thickening agent may be selected from the group consisting of carboxymethylcellulose, microcrystalline cellulose, hypromellose (hydroxypropyl methylcellulose), hydroxypropylcellulose and mixtures thereof, xanthan gum, carrageenan, locust bean gum or guar gum, preferably carboxymethylcellulose, microcrystalline cellulose, and mixtures thereof and most preferably carboxymethylcellulose. Other suitable thickeners include Colloidal microcrystalline cellulose (commercially available as Avicel^{®} RC 591, Avicel^{®} CL 611 and Avicel^{®} BV 2219 from the FMC Corporation), Carbomer homopolymer Type A (commercially available as Carbopol^{®} 971 from the Lubrizol corporation), Carbomer homopolymer Type B (commercially available as Carbopol^{®} 974 from the Lubrizol Corporation).

The surfactant could be at least one poloxamer, such as poloxamer 188, poloxamer 338, poloxamer 237 and poloxamer 407, preferably poloxamer-188.

The pharmaceutical liquid composition may further comprise at least one active ingredient as defined above. Examples of active ingredient are decongestants, antihistamines, mucolytics, analgesics or antacids.

Other suitable pharmaceutical active ingredients include analgesics, anti-inflammatory agents, antiarthritics, anesthetics, antihistamines, antitussives, antibiotics, anti-infective agents, antipyretics, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, cough suppressants, decongestants, expectorants, oral contraceptives, diuretics, gastrointestinal agents, migraine preparations, motion sickness products, mucolytics, muscle relaxants, osteoporosis preparations, polydimethylsiloxanes, respiratory agents, sleep-aids, urinary tract agents, and pharmaceutically acceptable salts thereof, derivatives thereof, combinations thereof and mixtures thereof.

In accordance with an embodiment, the active ingredient is selected from acetyl salicylic acid, acetic acid derivatives such as indomethacin, diclofenac, sulindac, and tolmetin; fenamic acid derivatives such as mefanamic acid, meclofenamic acid, and flufenamic acid; biphenylcarbodylic acid derivatives such as diflunisal and flufenisal; and oxicams such as piroxicam, sudoxicam, isoxicam, and meloxicam; and pharmaceutically acceptable salts thereof, derivatives thereof, combinations thereof and mixtures thereof.

Examples of useful NSAIDs include ibuprofen, naproxen, benoxaprofen, naproxen sodium, fenbufen, flurbiprofen, fenoprofen, fenbuprofen, ibuprofen, ketoprofen, indoprofen, pirprofen, carpofen, oxaprofen, pranoprofen, microprofen, tioxaprofen, suprofen, alminoprofen, tiaprofenic acid, fluprofen, bucloxic acid, celecoxib, and pharmaceutically acceptable salts thereof, derivatives thereof, combinations thereof and mixtures thereof.

Examples of cough and cold pharmaceutical active ingredients, which include antihistamines, cough suppressants, decongestants. Demulcents, such as pectin and expectorants, include, but are not limited to, bromopheniramine, carbinoxamine, acetylcysteine, guaifenesin, carbocysteine, chlorcyclizine, dexbrompheniramine, bromhexane, phenindamine, pheniramine, pyrilamine, thonzylamine, pripolidine, ephedrine, phenylephrine, pseudoephedrine, phenylpropanolamine, chlorpheniramine, dextromethorphan, diphenhydramine, doxylamine, astemizole, terfenadine, fexofenadine, naphazoline, oxymetazoline, montelukast, propylhexadrine, triprolidine, clemastine, acrivastine, promethazine, oxomemazine, mequitazine, buclizine, bromhexine, ketotifen, terfenadine, ebastine, oxatamide, xylomeazoline, loratadine, desloratadine, noscapine, clophedianol, menthol, benzonatate, ethylmorphone, codeine, acetylcysteine, carbocisteine, ambroxol, belladona alkaloids, sobrenol, guaiacol and cetirizine; and pharmaceutically acceptable salts thereof, derivatives thereof, combinations thereof and mixtures thereof.

In another embodiment, the at least one active ingredient is an NSAID and/or acetaminophen, and pharmaceutically acceptable salts thereof.

In an embodiment, the active ingredient is a topical analgesic such as benzocaine, benzydamine, dexpanthenol, menthol flurbiprofen or diclonene.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotidine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate, loperamide and racecadotril; glycopyrrolate; antiemetics, such as ondansetron, analgesics, such as mesalamine. In one embodiment the composition of the present invention is present in an antacid containing suspension. In another embodiment, the composition contains a therapeutic ingredient such as a natural. Natural therapeutic ingredients may include but are not limited to honey, ivy, peppermint, elder, sage, thyme, or lemon balm.

In some embodiments, a preservative component may be used. Such a preservative component may be selected from any pharmaceutically acceptable preservative. The alkyl esters of para-hydroxybenzoic acid (the parabens, e.g., butylparaben, methylparaben and propylparaben) are examples and may be used alone or in combination. Generally, the parabens are used in a concentration of about 0.02% w/w. Other preservatives include ethylenediamine tetra-acetic acid, propyl-p-hydroxybenzoates, antioxidants or sorbic acid.

The compositions may also contain colorants and/or sweeteners as appropriate. The sweetening agents may be for example bulk sweeteners such as sugars (e.g., sucrose or fructose) or polyols (e.g., maltitol, xylitol, sorbitol, sucralose) and/or intense sweeteners such as saccharin, aspartame or acesulfame K.

The ratios of the technical features including cooling agent, surfactant and/or viscosity enhancing agent are shown in Table 1.

**Table 1.**

| Ingredients | Ratio |
|---|---|
| Cooling agent : surfactant | 0.4-0.01 : 0.4-0.1 |
| Surfactant : viscosity enhancing agent | 1.0-0.5 : 1.0-5.0 |
| Cooling agent : surfactant : viscosity enhancing agent | 1 : 2 : 4 |

The pharmaceutical liquid composition comprises the viscosity enhancing agent in an amount of about 0.1 %w/w to about 5 %w/w, the surfactant in an amount of about 0.1 %w/w to about 1.0 %w/w and the cooling agent in an amount of about 0.005 %w/w to about 1.0% %w/w relative to the pharmaceutical liquid composition.

In one embodiment, the pharmaceutical liquid composition comprises carboxymethylcellulose in an amount of from 0.1 %w/w to 1.0 %w/w, Poloxamer-188 in an amount of from 0.1 %w/w to 0.5 %w/w and the menthyl glutarate in an amount of from 0.01 %w/w percent to 1.0 %w/w percent relative to the pharmaceutical liquid composition.

Finally, the invention relates to a method of alleviating a symptom selected from the group consisting of cough, nasal congestion, allergy and sore throat in a subject comprising administering the pharmaceutical liquid composition as defined above, such as sore throat.

### Examples

### Materials

Avicel^{®} (Microcrystalline Cellulose) were purchased from the FMC Corporation.

CMC (Carboxymethylcellulose) were purchased from the Ashland Chemical Corporation.

HPMC (Hypromellose) were purchased from the Ashland Chemical Corporation.

Mucin were purchased from the Sigma Aldrich Corporation.

Poloxamer-188 were purchased from the BASF Corporation.

Cooling agents were purchased from the International Fragrances and Flavors Corporation (IFF), Takasago Corporation, the Firmenich Corporation, the Symrise Corporation and the Givudan Corporation.

### Example 1: Mixture of various materials for determining Zeta-Potential

Various polymeric additives were used as rheology and texture modifiers to improve the mouth-feel experiences of cough-cold liquid formulations. These additives included various grades of Avicel^{®} (Microcrystalline Cellulose), CMC (Carboxymethylcellulose), and HPMC (Hypromellose). A synergistic effect was observed when a poloxamer was also included in the formulation containing above mentioned rheology modifier and a cooling flavor. A cooling sensation in the back of the throat could be felt, and the sensation lasted longer (approximately 5-10 mins or even longer in some instances). Additional studies were carried out to understand the effect, and it was discovered that, the targeting effect and longer lasting sensation were not present in low viscosity liquids even when the poloxamer was included in the formulation. In presence of just the viscosity modifiers without a poloxamer, the same flavor did provide targeted cooling, but the extent and duration was less.

To better understand the mechanism behind targeted longer lasting cooling experienced in samples containing rheology modifier (Avicel, CMC), Poloxamer and Cooler # 2, the following studies were carried out:

### Zeta Potential measurement:

The zeta potential (ζ-potential) is the potential difference across phase boundaries between solids and liquids. It is a measure of the electrical charge of particles that are suspended in liquid. Upon addition of polymer solution to mucin suspension, a change in the zeta potential of mucin particle may be indicative of Polymer-mucin interaction (electrostatic double formation).

Mucin from porcine stomach was used for mucoadhesion studies. Briefly, mucin was suspended in deionized water and mixed overnight using a magnetic stirrer to completely hydrate and suspend mucin. CMC and Poloxamer-188 were separately dissolved in deionized water at 0.5% level. Using a Malvern Zetasizer Nano, the zeta potential of 0.5 wt% mucin was determined in triplicate. Initial experiments were carried out at different mucin level of mucin in suspension (0.1wt% to 1.0wt%) and reproducible zeta potential from separate mucin suspension preparation could be obtained at 0.5 wt% level. To measure mucin-polymer interaction, different amounts of a polymer solution (0.5 wt% in water) were added to mucin suspension and the mixture was incubated at 37°C for 15 min before measuring zeta potential. The measured zeta potential of 0.5wt % porcine mucin suspension in water was -4.5 mV. Upon addition of CMC or Poloxamer-188 solution to mucin suspension, the zeta potential of the mucin particle changed to a more negative value as shown in Table 2.

**Table 2: Zeta Potential of Mucin Particle in presence of CMC and Poloxamer-188**

| Sample | Mucin : Polymer ratio | Zeta Potential (mV) |
|---|---|---|
| Mucin only | 1: 0 | -4.563 ±0.5096 |
| Mucin- CMC | 1:1 | -16.6 ± 0.4582 |
| | 1:10 | -39.47 ± 1.1590 |
| | 1:20 | -43.2 ± 2.2068 |
| Mucin- Poloxamer-188 | 1:1 | -6.7 ± 0.6885 |
| | 1:10 | -13.8 ± 0.6083 |
| | 1:20 | -12.87 ± 1.9757 |
| Mucin-CMC-Poloxamer | 1:10:20 | -42.7 ± 1.6092 |

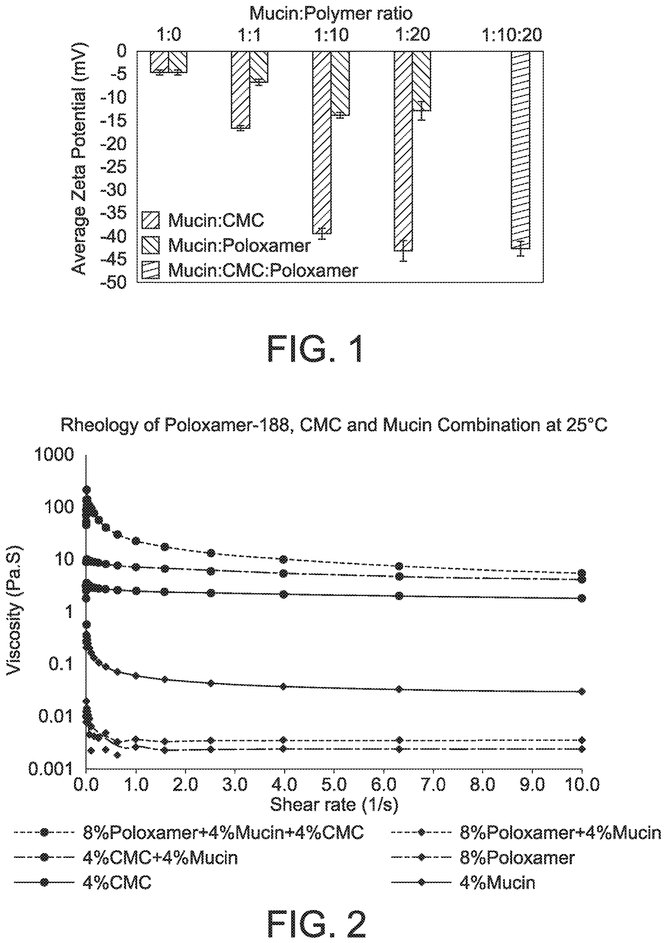

Different amounts of CMC and Poloxamer-188 solution were added to a known volume of mucin suspension, and it was noticed that the zeta potential of Mucin-CMC and Mucin-Poloxamer system shifted to a higher negative value initially as the polymer level was increased, reaching a plateau at 1:10 Mucin : CMC ratio and Mucin : Poloxamer-188 ratio. This shifting of zeta potential to a higher negative value is indicative of the interaction between polymer and mucin. In order to see if Poloxamer and CMC combined have a synergistic effect, both Poloxamer and CMC solutions were added to Mucin suspension to get a 1:10:20 ratio of Mucin : CMC : Poloxamer (CMC to Poloxamer ratio in cough cold liquids is ~1:2). A small increase in the zeta potential from -39.47 mV (for Mucin:CMC 1:10) to 42.7mV was observed (Fig. 1).

CMC is an anionic polymer and like other anionic mucoadhesive polymers such as Carbopol, it is expected that the zeta potential of mucin will shift to a higher negative value. However, Poloxamer-188 is a non-ionic polymer and electrostatic interaction are not expected to be present in this system. Poloxamers are block copolymers consisting of central hydrophobic chain of polyoxypropylene attached to two hydrophilic chains of polyoxyethylene. Literatures suggest that Poloxamers have mucoadhesive properties owing to its hydrogen bonding capabilities. Looking at the structure, Poloxamer would not be a hydrogen bond donor since it does not have -OH group in it (hydrogen atom covalently bonded to one of electronegative oxygen atoms). However, due to the presence of 2 lone pair of electrons over the oxygen atom of the oxyethylene part of the molecule, poloxamer molecules in aqueous environment can acquire δ-ve charge and have ability to form a hydrogen bond with a hydrogen bond donor. Presence of this δ-ve charge on the poloxamer molecule, in presence of a hydrogen bond donor would make a Poloxamer act like a weak anionic polymer and possibly cause the zeta potential of Mucin-Poloxamer system shifts to a higher -ve value when mixed together.

### Example 2: Rheology Studies:

Rheological studies of mucin, polymer and its blends were carried out with an intent to understand synergism and mucoadhesion. Solutions of 8% Poloxamer-188 and 4% CMC were prepared by separately dissolving Poloxamer-188 and CMC in purified water. A 4% Mucin suspension was prepared by mixing mucin in purified water overnight using an overhead mixer.

Rheology data for (steady state shear sweep) for 8% Poloxamer-188 solution, 4% CMC-solution and 4% Mucin suspension was measured. For the interaction study, a known amount of Poloxamer was dissolved in 4% Mucin suspension to obtain an 8% Poloxamer-188 in 4% Mucin suspension. CMC-Mucin mixture was prepared in a similar way by dissolving 4% CMC in a 4% Mucin suspension. Steady state shear sweep curves for Poloxamer-188 solution, CMC solution Mucin suspension and combination of Mucin-Poloxamer, Mucin-CMC and Mucin-Poloxamer-CMC at 25°C are presented in Fig. 2. These curves were analyzed using a TA Instruments AR 2000 rheometer used in a parallel plate configuration.

For this study high levels of polymer (Poloxamer-188 and CMC) were used to magnify the effect of interaction between Mucin and polymers (Poloxamer-188 and CMC). As seen in figure 2, Poloxamer-188 has the lowest viscosity (approximately 0.0027 Pa.S at a shear rate of 1.00 1/s), at the same shear rate, the viscosity of mucin suspension is approximately 0.06113 Pa.S. However, a significant drop in the viscosity can be observed for Mucin and Poloxamer-188 mixture where both components are present at the same level. This reduction in viscosity has not been previously seen or reported.

Viscosity of 4% CMC solution at 1.00 1/s shear rate is approximately 2.545 Pa.S and combination of Mucin-CMC system at the same shear rate has a viscosity of 7.274 Pa.S and Mucin-CMC-Poloxamer-188 is 23.1 Pa.S. If 2 non-interacting polymers are mixed together, the final viscosity is expected to be a combination of the polymers dissolved with viscosity primarily controlled by the component with highest viscosity. However, in presence of synergistic interaction, the final viscosity would be significant higher or lower as seen in case of Mucin-CMC, Mucin-Poloxamer and Mucin-CMC-Poloxamer.

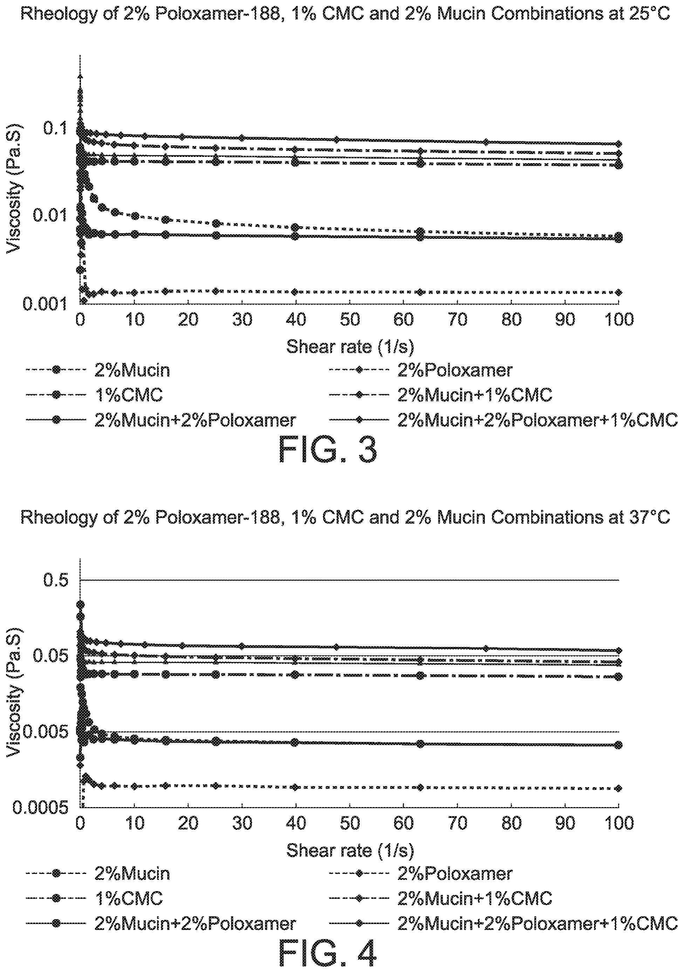

Since significantly higher concentrations of Poloxamer-188 and CMC were used, the study was repeated using lower concentrations of Poloxamer-188 and CMC. Rheological measurements were carried out at both 25°C and 37°C. Figs. 3 and 4 shows the rheological profile for 2% Poloxamer-188, 1%CMC, 2% Mucin and their combinations.

As seen in Figs. 3 and 4, a similar trend in the viscosity profile can be seen at both 25°C and 37°C where the viscosity of Mucin-CMC and Mucin-CMC-Poloxamer system is higher than the additive viscosities of the components.

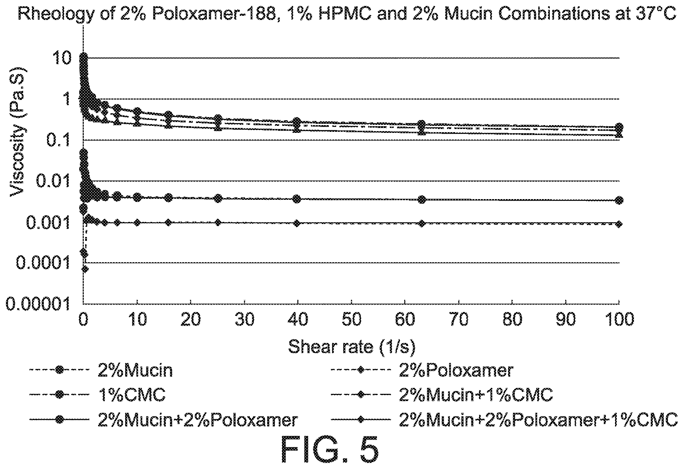

As discussed previously, if a non-interacting polymer is mixed together with Mucin, a synergism is not expected and a significant change in the viscosity is not expected. To evaluate this, studies were also carried out using a non-ionic polymer (HPMC) instead of CMC. Rheology results for 2% Poloxamer-188, 1% HPMC E4M and 2% Mucin combination at 37°C are presented in Fig. 5. Absence of interaction between HPMC, Mucin and Poloxamer can clearly be seen from the graph since the HPMC curve almost overlaps with HPMC + Mucin + Poloxamer curve or HPMC+ Mucin curve. Since the measurement was done at 37°C, the degree of reduction in viscosity of Mucin-Poloxamer system was negligible as seen previously.

### Example 3: Chemical Interaction between the Cooling sensate and Polymer by FTIR and ¹H NMR:

If a chemical interaction like hydrogen bonding exists between the sensate (cooler #2) and mucoadhesive polymer (Poloxamer-188 and/or CMC), the cooling sensation due to the sensate will be longer, since the sensate would preferentially be distributed in the polymer phase, adhering to the mucosal membrane and provide cooling. To identify the presence of hydrogen bonding between Cooler # 2 and Poloxamer-188 and/or CMC, FTIR spectrum of mixture of Cooler # 2 with Poloxamer-188 and CMC were compared to individual components, and no peak shifting or changes in peak shape could be observed, indicating the absence of any chemical interaction between them.

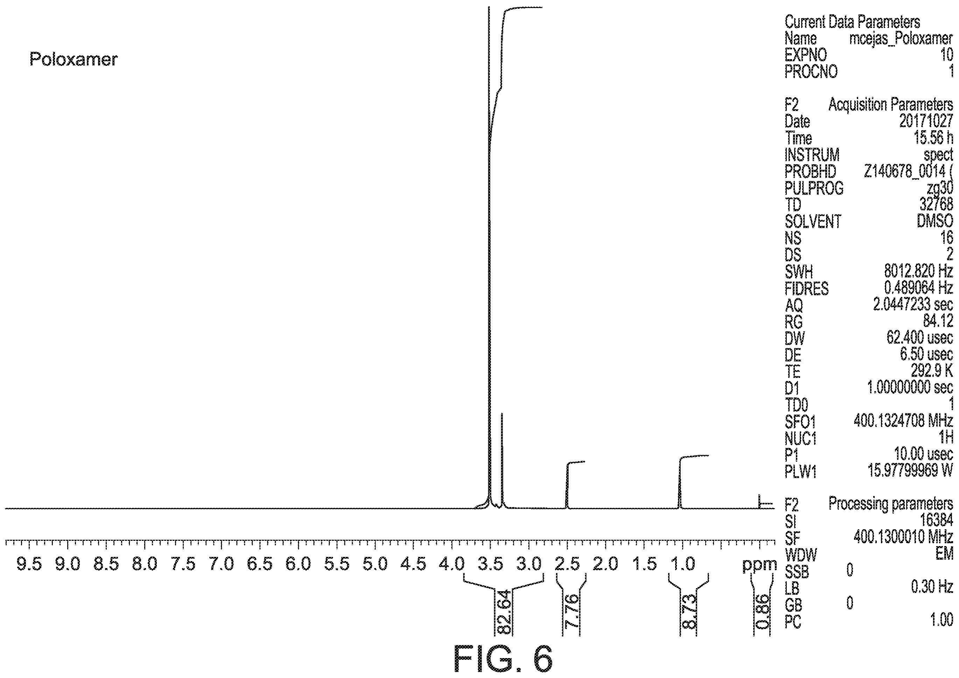

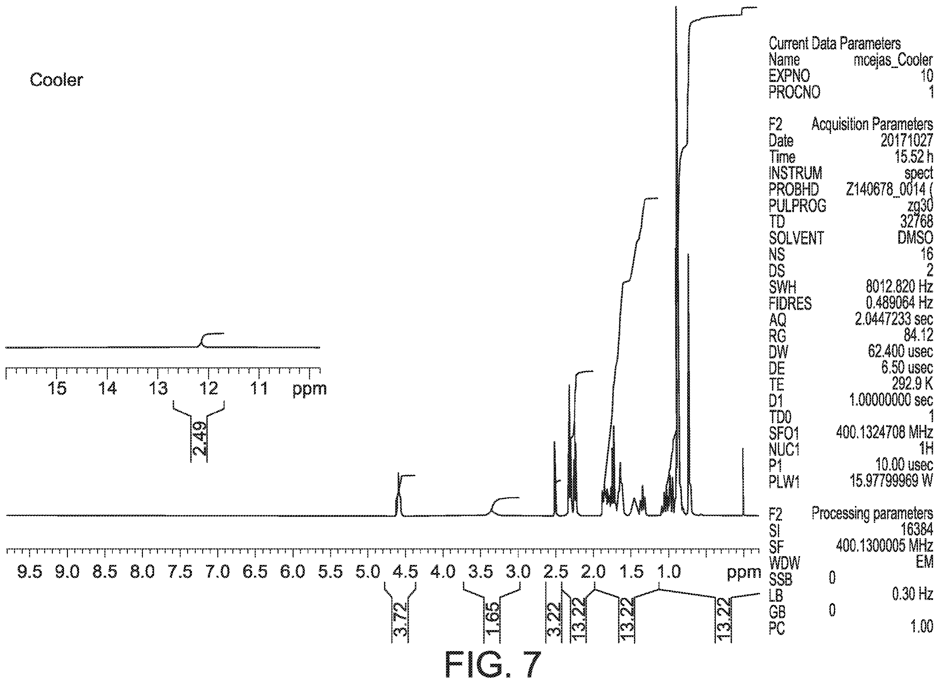

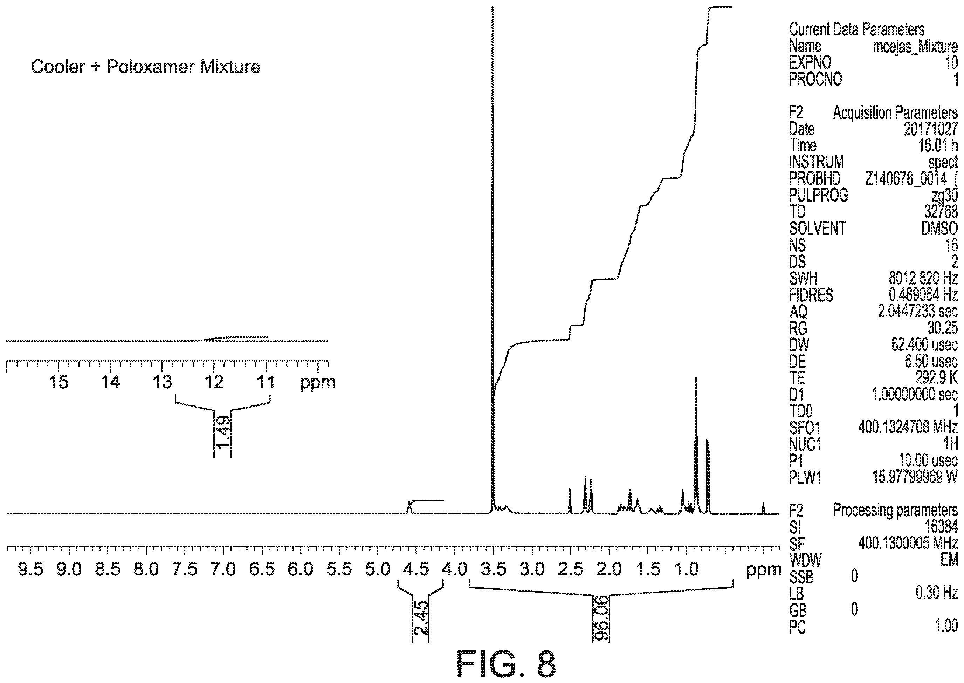

Proton NMR spectrum of Poloxamer-188, Cooler # 2 and a mixture of Cooler # 2 and Poloxamer-188 was acquired by dissolving both them in deuterated DMSO. Figure 6,7 and 8 are ¹H NMR of Poloxamer-188, Cooler #2 and mixture of Poloxamer-188 and Cooler # 2 in deuterated DMSO respectively.

### Example 4: Results and Summary of Examples 1-3:

Based on the available rheology and zeta potential measurement data, it is clear that some degree of interaction exists between Mucin and CMC. This interaction could be attributed to electrostatic interaction and/or chain entanglement between mucin and CMC similar to other anionic mucoadhesive polymers like Carbopols. In presence of non ionic polymer like HPMC no interaction could be observed.

Interaction between mucin and Poloxamer-188 was also evident from rheology and zeta potential measurements. Presence of Poloxamer resulted in reduction in viscosity of Mucin suspension. Zeta potential measurement also indicated interaction but to a lesser degree compared to CMC. Rheology data from combination of Mucin-CMC-Poloxamer at both low and high concentration and temperature showed significant increase in the viscosity that far exceeds the additive effect. Based on the available data it can be theorized that the viscosity of the cough cold liquid increases significantly when it comes in contact with the mucosal tissue in the oral cavity (as it is being swallowed). Due to this increase in the viscosity, the residence time of the liquid will increase, creating a coating effect in the throat area, and providing longer lasting cooling sensation in the throat area due to the presence of Cooler # 2.

Absence of peak shifting in FTIR could be attributed to absence of liquid (aqueous) media that can promote hydrogen bonding. It should be noted that if the 2 components are mixed together in an aqueous media, the hydrogen bonding peaks will be completely masked/covered by water peak and hence, FTIR would probably not be the best tool. ¹H NMR data did not show any indication of existence of chemical interaction like hydrogen bonding between Poloxamer and Cooler # 2. However, it is worth noting that the measurement was carried out in d-DMSO (Cooler # 2 is insoluble in water and hence d-DMSO was used) and under these conditions hydrogen bonding may be absent.

### Example 5: Taste-Test Evaluation

Various polymeric additives were combined into a base cough-cold liquid formula to determine interaction, and included various grades of Avicel^{®}, CMC, HPMC among others. The base formula is shown in Table 6. A synergistic effect was observed when Poloxamer was also included in the formulation containing above mentioned rheology modifier and a cooling flavor. A cooling sensation in the back of the throat could be felt, and the sensation lasted longer (approximately 5-10 min or even longer in some instances). Additional studies showed that the targeting effect and longer lasting sensation was not present in low viscosity liquids even when Poloxamer was included in the formulation. The presence of just the viscosity modifiers and no Poloxamer, the same flavor did provide targeted cooling, but the extent and duration was less.

Additional studies were carried out to understand the effect of flavor/sensate in formulations containing both Poloxamer and viscosity modifiers.

Cooling sensate samples were requested from various flavor suppliers and were included into the formulation at a level recommended by the manufacturer and evaluated by various individuals in terms of cooling efficiency/intensity, area within the oral cavity where cooling could be felt, cooling onset time, and duration of cooling. It was interesting to see that all the sensate provided different degree of cooling and the area within the oral cavity where cooling could be felt was different. Majority of the sensate provided cooling in the mouth, tongue or palate with minimal to no cooling in the back of the throat except for Cooler # 2, Symcool^{®} NAT, WS-3 and WS-5. Cooler #2 did not have any cooling in the mouth and all the cooling was in the back of the throat, however other 3 provided cooling in mouth, tongue along with some cooling in the back of the throat. Below is a summary of taste evaluation of various sensate systems (also shown in Table 3).

Taste Evaluation results:
1. Takasago Intensates^{®} Morning Blast J75 (TAK-171454), addition level (0.01%):
   a. Cooling Intensity: Mildly cooling with tingling sensation;
   b. Cooling site: Mostly tongue and some towards back of mouth;
   c. Onset time: less than 1 min;
   d. Duration: 2-3 minutes;
   e. Overall: Not noticeable.
2. Takasago Intensates^{®} Fire & Ice C83 (TAK-171456), addition level (0.05%):
   a. Cooling Intensity: Warming with tingling sensation;
   b. Cooling site: mostly on tongue;
   c. Onset time: less than 1 min (Immediate warming);
   d. Duration: 2-3 minutes of warming;
   e. Overall: Cooling not noticeable, warming sensation.
3. Takasago Intensates^{®} Triple Cool L92 (TAK-171458), addition level (0.05%):
   a. Cooling Intensity: Tingling sensation, very low level cooling, less warming compared to TAK-171456;
   b. Cooling site: mostly on tongue;
   c. Onset time: slightly delayed @ 1 min;
   d. Duration: 2-3 min;
   e. Overall: Cooling not noticeable, tingling sensation only.
4. Firmenich Art Freeze Flavor (329816 02602TH), addition level (0.3%):
   a. Cooling Intensity: Tingling sensation, low level cooling, cooling intensity grows with time;
   b. Cooling site: Mostly on tongue, front of the mouth, and over all mouth cavity, no throat cooling;
   c. Onset time: slightly delayed @ 1-2 min;
   d. Duration: >10 mins;
   e. Overall: Cooling noticeable all around the oral cavity, tongue, and lips, no cooling in throat.
5. Firmenich Novasense^{®} Cool Flavor (534751 T), addition level (0.012%):
   a. Cooling Intensity: low level cooling, cooling intensity less than Art Freeze, with menthol notes;
   b. Cooling site: mostly on tongue, front of the mouth, no throat cooling;
   c. Onset time: Immediate ~10 secs;
   d. Duration: >4-5 mins;
   e. Overall: Cooling noticeable all around the oral cavity, tongue, and lips, no cooling in throat.
6. Symrise Symcool^{®}WS-5, addition level (0.01%)*:
   a. Cooling Intensity: Intense Cooling;
   b. Cooling site: towards the back on mouth, upper throat, and tongue;
   c. Onset time: Immediate ~10 secs;
   d. Duration: >6-7 mins;
   e. Overall: Intense cooling noticeable all around the oral cavity, tongue, and upper throat.
   ***Did work, providing synergistic sensate effect**
7. Symrise Symcool^{®}WS-3, addition level (0.025%)*:
   a. Cooling Intensity: Intense Cooling, more intense than WS-5;
   b. Cooling site: towards the back on mouth, upper throat, and tongue;
   c. Onset time: Immediate ~10 secs;
   d. Duration: >4-5 mins;
   e. Overall: Intense cooling noticeable all around, tongue, and upper throat, sweeter sensation like menthol.
   ***Did work, providing synergistic sensate effect**
8. Symrise Symcool^{®}WS-23, addition level (0.03%):
   a. Cooling Intensity: Mild Cooling, less than WS-3 and WS-5;
   b. Cooling site: tongue and front of the mouth;
   c. Onset time: Immediate ~10 secs;
   d. Duration: >3-4 mins;
   e. Overall: Mild cooling noticeable on tongue, and front of the mouth, tingling and nose cooling as you breath out.
9. Symrise Symcool^{®}WS-500, addition level (0.04%):
   a. Cooling Intensity: Intense Cooling, similar to WS-3;
   b. Cooling site: tongue and upper throat;
   c. Onset time: Immediate ~10 secs;
   d. Duration: >3-4 mins;
   e. Overall: Intense cooling noticeable in front of the mouth and throat, with bitter off notes.
10. Symrise Symcool^{®}WS-12, addition level (0.004%):
   a. Cooling Intensity: Mild Cooling;
   b. Cooling site: mostly tongue and some in upper throat;
   c. Onset time: slow onset 1-2 min;
   d. Duration: >5-7 mins;
   e. Overall: Mild cooling noticeable on tongue and some back of the throat.
11. Symrise OPTACOOL^{®} T (295695), addition level (0.03%):
   a. Cooling Intensity: Intense Cooling;
   b. Cooling site: mostly tongue, and front of the mouth;
   c. Onset time: Immediate;
   d. Duration: >5-7 mins;
   e. Overall: Intense cooling noticeable on tongue and front of the mouth. Bitterness.
12. Symrise OPTACOOL^{®} A (291894), addition level (0.03%):
   a. Cooling Intensity: Less cooling compared to Optacool T;
   b. Cooling site: mostly tongue, and front of the mouth, no throat;
   c. Onset time: Immediate;
   d. Duration: >5-7 mins;
   e. Overall: Cooling noticeable on tongue and front of the mouth. More bitterness than Optacool T.
13. L-Menthol, addition level (0.01%):
   a. Cooling Intensity: Intense cooling;
   b. Cooling site: Throat and some in oral cavity tongue and front of the mouth;
   c. Onset time: Immediate;
   d. Duration: >5-6 mins;
   e. Overall: Cooling noticeable on tongue and front of the mouth and throat.
14. Combination Symcool^{®}WS-5 (0.005%) Symcool^{®}WS-3 (0.0125%) L-Menthol (0.005%)*:
   a. Cooling Intensity: Intense cooling;
   b. Cooling site: Throat and some in oral cavity tongue and front of the mouth;
   c. Onset time: Immediate;
   d. Duration: >5-7 mins;
   e. Overall: Cooling noticeable towards the back of the throat, on tongue and front of the mouth.
   * **Did work, providing synergistic sensate effect**
15. IFF cooler # 2 addition level (0.1%)*:
   a. Cooling Intensity: Mild to medium cooling;
   b. Cooling site: Throat;
   c. Onset time: Slightly delayed ~30 secs;
   d. Duration: >7-9 mins;
   e. Overall: Cooling concentrated towards the back of the throat, intensity grows with time and duration longer than others ~7-9 mins.
   * **Did work, providing synergistic sensate effect**
16. Symrise Symcool^{®} NAT addition level (0.05%)*:
   a. Cooling Intensity: medium cooling;
   b. Cooling site: Tongue and some throat cooling;
   c. Onset time: immediate;
   d. Duration: >5-6 mins;
   e. Overall: Liquorish taste and cooling in the mouth and towards the back of the throat, cooling in throat as you breath in, intensity grows with time.
   ***Did work, providing synergistic sensate effect**
17. Givaudan Evercool^{®} L-034206, addition level (0.05%)
   a. Cooling Intensity: medium cooling;
   b. Cooling site: Mostly Tongue and roof of the mouth, sweeter taste;
   c. Onset time: immediate;
   d. Duration: >2-3 mins;
   e. Overall: Cooling in the tongue and roof of the mouth, felt like coating on the roof of the mouth, nothing in the front of the mouth like lips etc.
18. Givaudan Evercool^{®} S-018144, addition level (0.05%):
   a. Cooling Intensity: low to medium cooling;
   b. Cooling site: Mostly Tongue and roof of the mouth, sweeter taste;
   c. Onset time: slightly delayed ~15-30 secs;
   d. Duration: >2-3 mins;
   e. Overall: Cooling in the tongue and roof of the mouth, milder than Evercool Liquid, cooling sensation faded away faster than Evercool liquid.

**Table 3: Summary of Taste Evaluation:**

| **Manufacturer** | **Sensate or System** | **Area Targeted** | **Onset Time** | **Duration** | **Overall** |
|---|---|---|---|---|---|
| Takasago | Intensates^{®} Morning Blast J75 TAK-171454 (0.01%) | Mild cooling with tingling sensation | <1 min | 2-3 mins | Mostly tongue some in upper throat region, no significant cooling |
| | Intensates^{®} Fire & Ice C83 TAK-171456 (0.05%) | Warming with tingling mostly on tongue and some towards back of mouth | <1 min, immediate warming | 2-3 mins | Cooling not noticeable |
| | Intensates^{®} Triple Cool L92 TAK-171458 (0.05%) | Tingling sensation, with very low level cooling mostly on tongue | slightly delayed ~1 min | 2-3 mins | Cooling not noticeable |
| Firmenich N/A | Art Freeze Flavor 329816 02602TH (0.3%) | Tingling sensation, low level cooling, cooling intensity grows with time. Mostly on tongue and palate | delayed 1-2 min | >10 mins | Mild cooling initially that grows with time. Cooling felt in mouth, including lips nothing in the back of the throat. |
| | Novasense Cool Flavor 534751 T (0.012%) | Low level cooling, overall intensity less than Art Freeze, mostly on tongue and front of the mouth and lips | Immediate ~10 secs | 4-5 mins | Milder cooling, all over the oral cavity including lips, not throat cooling |
| | L-Menthol (0.01%) | Intense cooling in throat and some in oral cavity tongue and front of the mouth | Immediate ~10 secs | 5-6 mins | Cooling noticeable on tongue and front of the mouth and throat |
| | Symcool^{®}WS-5 (0.01%) | Intense cooling in tongue, back of the mouth and throat (upper) | Immediate ~10 secs | 6-7 mins | Intense cooling noticeable all around the oral cavity, tongue, and upper throat |
| | Symcool^{®}WS-3 (0.025%) | Intense cooling in tongue, back of the mouth and throat (upper). More intense than WS-5 | Immediate ~10 secs | 4-5 mins | Intense cooling noticeable all around, tongue, and upper throat, sweeter sensation. |
| Symrise | Symcool^{®}WS-23 (0.03%) | Mild cooling, less than WS-3 and WS-5, mostly on tongue and front of the mouth | Immediate ~10 secs | 3-4 mins | Mild cooling noticeable on tongue, and front of the mouth, tingling and nose cooling as you breathe out. |
| | Symcool^{®}WSE-500 (0.04%) | Intense cooling similar to WS-3 on tongue and upper throat | Immediate ~10 secs | 3-4 mins | Intense cooling noticeable in front of the mouth and throat, with bitter off notes |
| | Symcool^{®}WS-12 (0.004%) | Mild cooling mostly tongue and some in upper throat | slow onset 1-2 min | 5-7 mins | Mild cooling noticeable on tongue and some back of the throat |
| | 295695 OPTACOOL^{®}T (0.03%) | Intense cooling, mostly tongue, and front of the mouth | Immediate ~10 secs | 5-7 mins | Intense cooling noticeable on tongue and front of the mouth. Bitterness |
| | 291894 OPTACOOL^{®}A (0.03%) | Less cooling compared to Optacool T, mostly tongue, and front of the mouth, no throat | Immediate ~10 secs | 5-7 mins | Cooling noticeable on tongue and front of the mouth. More bitterness than Optacool T. |
| | Symcool NAT (0.05%) | Medium intensity cooling in mostly in tongue and some cooling in the back of the throat | Immediate ~10 secs | 5-6 mins | Medium intensity liquorish taste with cooling on tongue and also in the back of the throat. Cooling intensity increases with time. Feel cooling as you breath in |
| Symrise (mix) | Symcool^{®}WS-5 (0.005%) Symcool^{®}WS-3 (0.0135%) L-Menthol (0.005%) | Intense cooling in "Throat and some in oral cavity tongue and front of the mouth | Immediate ~10 secs | 5-7 mins | Cooling noticeable towards the back of the throat, on tongue and front of the mouth. |
| Givaudan | Evercool L-034206 (0.05%) | Medium intensity cooling in the tongue and roof of the mouth, sweeter taste | Immediate ~10 secs | 2-3 mins | Cooling in the tongue and roof of the mouth, felt like coating on the roof of the mouth, nothing in the front of the mouth like lips etc. |
| | Evercool S-018144 (0.05%) | Mild to medium intensity cooling on tongue and roof of mouth, | Slightly delayed | ~ 2 mins | Cooling in the tongue and roof of the mouth, milder than Evercool Liquid, cooling sensation faded away faster than Evercool L034206. |

**Table 4: Base Cough Cold Liquid Placebo formulation**

| Materials | % w/w |
|---|---|
| Purified Water USP | 30.000% |
| Propylene Glycol USP | 14.000% |
| Microcrystalline cellulose and carboxymethylcellulose sodium (Avicel^{®} RC591¹) | 0.500% |
| Carboxymethylcellulose (CMC Aqualon^{®} 7M8SF PH)² | 0.250% |
| Kollisolv^{®} PEG 1450³ | 16.200% |
| Poloxamer-188, NF (Kolliphor^{®} P188³) | 0.400% |
| Glycerin USP | 7.400% |
| Anhydrous Citric Acid USP | 0.050% |
| Sodium Benzoate, NF | 0.200% |
| Sucralose, NF (Powder) | 0.150% |
| Sorbitol Solution USP 70% | 18.500% |
| Cooling Sensate (See taste-test evaluation results) | 0.01 to 0.1% |
| Purified Water USP (Qs) | 12.35% |
| FD&C Blue # 1 (Colorant) | 0.0015% |
| | |
| **Total** | **100.00%** |

| | |
|---|---|
| 1 Commercially available from the FMC Corporation 2 Commercially available from the Ashland Corporation 3 Commercially available from the BASF Corporation | |

The following sensate provided the most cooling towards the back of the throat.

Mixing Procedure: The following procedure was used to mix the materials in Table 3. The formulation was prepared by adding purified water to a suitable vessel. The Avicel RC591 was then added while mixing using a high shear mixer until dissolved, followed by addition of propylene glycol while mixing using a propeller mixer at about 500-1000 RPM. The rest of the ingredients were added and mixed at approximately 50-100 RPM for at least 30 minutes. After complete dissolution, the solution was heated using a water bath to a temperature between 140°-170° C for 15 minutes while continuously stirring. The solution was cooled to room temperature before adding propylene glycol while mixing. The rest of the ingredients were added and mixed for at least 30 minutes.

### Trade Name: Cooler # 2

Commercial Manufacturer, Source: International Flavors and Fragrances (IFF)
Chemical Name: Monomenthyl Glutarate
CAS #: 220621-22-7
Empirical formula: C15H26O4,
Molecular weight: 269.4g/ mole
Solubility: Ethanol and Propylene Glycol, insoluble in Water
Chemical Structure:

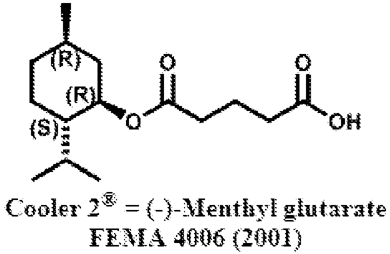

### Trade Name: Symcool NAT

Commercial Manufacturer, Source: Symrise
Chemical Name: Menthyl Lactate
CAS # 61597-98-6, 17162-29-7
Empirical Formula:C13H24O3,
Molecular weight: 228.4
Solubility: Propylene glycol, ethanol
Structure:

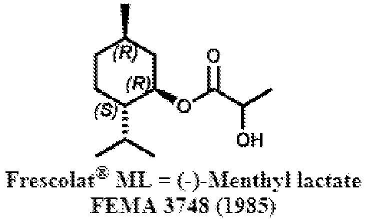

### Trade Name: WS -3

Commercial Manufacturer, Source: Symrise
Chemical Name: N-ethyl 2-isopropyl-5-methylcyclohexanecarboxamide CAS # 39711-79-0
Empirical Formula: C13 H25 N O
Molecular weight: 211.34 g/mol
Solubility: Propylene Glycol, ethanol, insoluble in water
Structure:

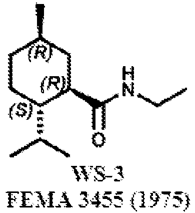

### Trade Name: WS-5

Source: Symrise
Chemical Name: N-[(ethoxycarbonyl)methyl)-p-menthane-3-carboxamide CAS # 68489-14-5
Empirical Formula: C15 H27 N O3
Molecular weight: 269.4 g/mol
Solubility: Propylene Glycol, ethanol, insoluble in water
Structure:

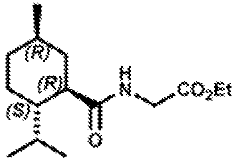

### Example 6: Conductivity Measurement

### Solution conductivity measurement:

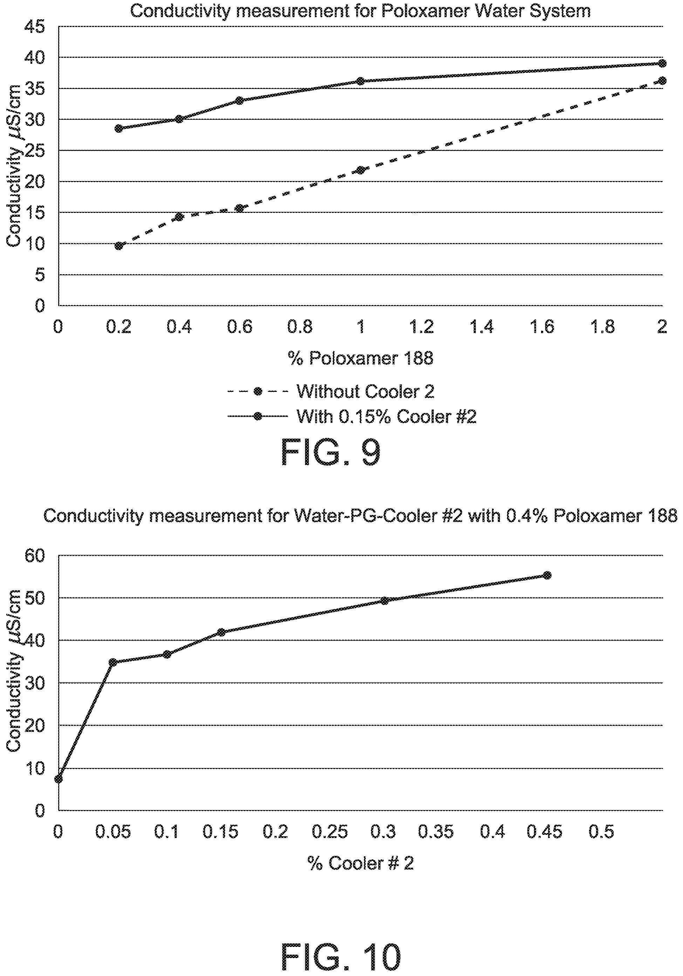

Conductivity of solutions containing Poloxamer-188 and Cooler # 2 in purified water at different concentrations was measured using a MultiLab 4010-2 conductivity meter with an IDS 4320 Conductivity and Temperature Sensor. Conductivity results are graphically presented in Fig.9.

The conductivity of the solution increases with increase in the concentration of Poloxamer-188 from 0.2% to 2.0%. Addition of 0.15% of Cooler # 2 has a significant effect on the conductivity of the solution at lower concentrations of Poloxamer-188, however the two curves seem to converge at higher levels of Poloxamer-188.

Since Cooler # 2 is insoluble in water, a Water-5% propylene glycol solution was used to prepare solutions containing different amount of Cooler #2 keeping Poloxamer concentration at 0.4%. The conductivity of the solution increases significantly from about 7.4µS/cm to about 35µS/cm by addition of just 0.05% of Cooler # 2. Increasing the level of Cooler #2 from 0.05% to 0.45% shows an almost liner increase from about 35µS/cm to about 55.3µS/cm (Fig. 2). It is important to note that the solutions turned cloudy once Cooler # 2 was added to the solution, indicating formation of an emulsion. The turbidity of the solutions was not very different as the level of Cooler # 2 was increased.

Increase in the conductivity of the solution could be indicative to increase in the ionic character of the solution and may contribute towards ionic attraction and binding with charged mucosal surface. Results are shown in Fig 10.

Conductivity of Solutions containing Viscosity modifiers (CMC and HPMC):
Conductivity of the Water-PG solutions containing 0.35% CMC and 0.35% HPMC was also measured in water and in combination with Cooler # 2 and Poloxamer. The conductivity results are summarized below in Table 5.

**Table 5.**

| Sample | Conductivity (µS/cm) |
|---|---|
| Purified water | 5.88 |
| 5 gm PG in 100 ml water | 6.56 |
| 0.35% CMC in 100 ml water | 559 |
| 0.35% HPMC E4M in 100 ml water | 18.2 |
| 95 ml (0.4% polox P188, 0.35% CMC & water sol +( 0.15 cooler-2 in 5 gm PG) | 446 |
| 95 ml (0.4% polox P188, 0.35% HPMC E4M & water sol +( 0.15 cooler-2 in 5 gm PG) | 40.9 |

Being an ionic polymer, the conductivity of 0.35% CMC solution is very high in water and also in presence of Poloxamer and Cooler # 2, however the addition of a non-ionic polymeric viscosity modifier HPMC E4M does not contribute much towards the conductivity of the solution. The conductivity of solution containing HPMC, Poloxamer 188 and Cooler #2 is almost same as the conductivity of solution at same level of Poloxamer 188 and Cooler # 2 without HPMC. Reduction in the conductivity of solutions containing HPMC would be indicative of less ionic character and not a significant ionic attraction and binding with charged mucosal surface. Similar trends were observed during rheology measurements where, when mixed with mucin suspension, change in the solution viscosity was less with HPMC compared to when CMC was used. The expected interaction with a mucosal surface (the throat) would be expected to be higher when using CMC due to the significant rise in conductivity.

### Table 6: Additional Cooling Evaluation

Coolers containing a phenyl ring and a side pendant group were evaluated for a throat cooling effect when compared to cooling agents that do not have these properties (e.g. "Cooler WS3" and "Menthol"), using the taste criteria shown in Example 6. Only the coolers with a phenyl ring and side pendant group displayed the positive targeted throat cooling effect.

| Cooling System/ Trade Name | Chemical Name/CAS # | Structure/ Molecular Weight | Targeted Throat Cooling |
|---|---|---|---|
| Cooler # 2 | Monomenthyl Glutarate | 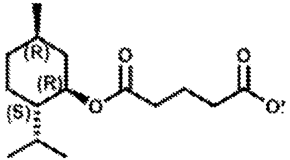 Molecular weight: 228.4 | Yes |
| | CAS Number: 220621-22-7 | | |
| Symcool NAT | Menthyl Lactate | 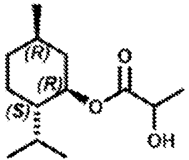 Molecular weight: 228.4 | Yes |
| | CAS Number: 61597-98-6, 17162-29-7 | | |
| WS -3 | N-ethyl 2-isopropyl-5-methylcyclohexanecarboxamide | 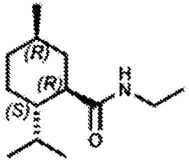 Molecular weight: 211.34 | Yes |
| | CAS Number: 39711-79-0 | | |
| WS-5 | N-[(ethoxycarbonyl)methyl)-p-menthane-3-carboxamide | 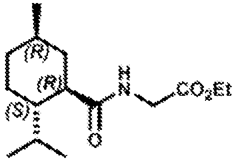 Molecular weight: 269.4 | Yes |
| | CAS Number: 68489-14-5 | | |
| WS 12 | (1R,2S,5R)-N-(4-methoxyphenyl)-5-methyl-2-(1-methylethyl)cyclohexanecarbox amide | 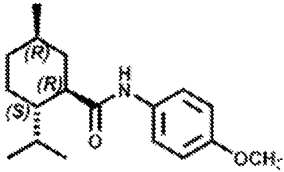 Molecular Weight: 289.4 | No |
| | CAS Number: 68489-09-8 | | |
| WS 23 | 2-isopropyl-N,2,3-trimethylbutyramide | 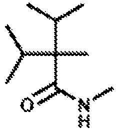 Molecular Weight: 171.28 | No |
| | CAS Number: 51115-67-4 | | |
| Menthol | (1R,2S,5R)-2-isopropyl-5-methylcyclohexanol | 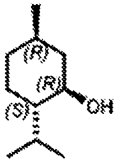 | No |
| | CAS Number: 89-78-1 | | |

### Example 7: Antacid Formulation Example

The following example was prepared using an antacid liquid in the composition of the present invention. The mixing procedure was the same as the one used for the base formulation in Table 3.

**Table 7: Antacid Formulation**

| Ingredient | Wt. (g) |
|---|---|
| Sorbitol Solution USP 70% | 18.0 |
| Microcrystalline cellulose and carboxymethylcellulose sodium (Avicel^{®} RC 591) | 0.5 |
| Simethicone Emulsion USP 30% | 0.5 |
| Magnesium Hydroxide, USP (5200 grade SPI) | 2.7 |
| Calcium Carbonate, USP (CalEssence 160 PCC Speciality Minerals)¹ | 8.0 |
| Sucralose | 0.1 |
| Artificial Cherry crème flavor # 56643 | 2.0 |
| Propylene Glycol | 1.5 |
| Poloxamer 188 | 0.4 |
| Cooler # 2 | 0.15 |
| Purified Water | QS to 100 g |

| | |
|---|---|
| 1: Commercially available from Specialty Minerals | |

The invention provides the following numbered aspects:
1. A pharmaceutical liquid composition, wherein said liquid composition comprises at least one cooling agent comprising of a phenyl ring containing a polar side group, at least one viscosity enhancing agent and at least one surfactant.
2. The pharmaceutical liquid composition of aspect 1, wherein the cooling agent is selected from the group consisting of menthyl glutarate, menthyl lactate, N-ethyl 2-isopropyl-5-methylcyclohexanecarboxamide, N-[(ethoxycarbonyl)methyl)-p-menthane-3-carboxamide and (1R,2S,5R)-N-(4-methoxyphenyl)-5-methyl-2-(1-methylethyl)cyclohexanecarboxamide.
3. The pharmaceutical liquid composition according to aspects 1 and 2, wherein the viscosity enhancing agent is selected from the group consisting of carboxymethylcellulose, microcrystalline cellulose, hypromellulose, hydroxypropylcellulose and mixtures thereof, xanthan gum, carrageenan, locust bean gum and guar gum.
4. The pharmaceutical liquid composition according to aspect 3, wherein the viscosity enhancing agent is selected from the group consisting of carboxymethylcellulose, microcrystalline cellulose, hypromellulose and mixtures thereof.
5. The pharmaceutical liquid composition according to aspect 3, wherein the viscosity enhancing agent is carboxymethylcellulose.
6. The pharmaceutical liquid composition according to aspects 1-5, wherein the surfactant is at least one poloxamer.
7. The pharmaceutical liquid composition according to aspect 6, wherein the poloxamer is poloxamer-188.
8. The pharmaceutical liquid composition according to aspect 7, wherein the active ingredient is selected from the group consisting of decongestants, antihistamines, mucolytics, expectorants, demulcents, analgesics and antacids.
9. A method of alleviating a symptom selected from the group consisting of cough, nasal congestion and sore throat in a subject comprising administering a pharmaceutical liquid composition according to any of preceding aspects.
10. The method of aspect 9, wherein the symptom is sore throat.

## Claims

1. A pharmaceutical liquid composition, wherein said liquid composition comprises:
at least one cooling agent selected from the group consisting of menthyl glutarate, menthyl lactate, N-ethyl 2-isopropyl-5-methylcyclohexanecarboxamide, and N-[(ethoxycarbonyl)methyl)-p-menthane-3-carboxamide,
at least one viscosity enhancing agent selected from the group consisting of carboxymethylcellulose, microcrystalline cellulose, hydroxypropylcellulose and mixtures thereof, carrageenan, locust bean gum and guar gum, and
at least one surfactant wherein the surfactant is at least one poloxamer,
wherein said liquid composition comprises the viscosity enhancing agent in an amount of 0.1 %w/w to 5 %w/w, the surfactant in an amount of 0.1 %w/w to 1.0 %w/w and the cooling agent in an amount of 0.005 %w/w to 1.0 %w/w relative to the pharmaceutical liquid composition,
wherein said liquid composition further comprises an active ingredient which is cetirizine.

2. The pharmaceutical liquid composition according to claim 1, wherein the viscosity enhancing agent is selected from the group consisting of carboxymethylcellulose, microcrystalline cellulose, and mixtures thereof.

3. The pharmaceutical liquid composition according to claim 2, wherein the viscosity enhancing agent is carboxymethylcellulose.

4. The pharmaceutical liquid composition according to claims 1-3, wherein the poloxamer is poloxamer-188.

5. The pharmaceutical liquid composition according to claims 1-4, wherein said liquid composition comprises carboxymethylcellulose in an amount of from 0.1 %w/w to 1.0 %w/w, Poloxamer-188 in an amount of from 0.1 % w/w to 0.5 %w/w and the menthyl glutarate in an amount of from 0.01 %w/w to 1.0 %w/w relative to the pharmaceutical liquid composition.

6. The pharmaceutical liquid composition according to any of the preceding claims, further comprising at least one further active ingredient selected from the group consisting of bromopheniramine, carbinoxamine, dexbrompheniramine, phenindamine, pheniramine, pyrilamine, thonzylamine, pripolidine, chlorpheniramine, diphenhydramine, doxylamine, astemizole, terfenadine, fexofenadine, triprolidine, clemastine, acrivastine, promethazine, oxomemazine, mequitazine, buclizine, ketotifen, terfenadine, ebastine, oxatamide, loratadine, desloratadine and pharmaceutically acceptable salts thereof, derivatives thereof, combinations thereof and mixtures thereof.
